# EUROPEAN PATENT SPECIFICATION

(11) **EP 4 482 424 B1**
(45) Date of publication and mention of the grant of the patent: **09.12.2026**
(21) Application number: 23723099.0
(22) Date of filing: 14.04.2023
(51) Int. Cl.: A61B 90/00

(54) **BIOPSY SITE MARKER WITH INCREASED VISUALIZATION AND NON-MIGRATION FEATURES**
BIOPSIESTELLENMARKER MIT ERHÖHTER VISUALISIERUNG UND NICHTMIGRATIONSEIGENSCHAFTEN
MARQUEUR DE SITE DE BIOPSIE PRÉSENTANT DES CARACTÉRISTIQUES DE VISUALISATION ET DE NON-MIGRATION ACCRUES

(30) Priority: 03.05.2022 US 202263337768 P
(43) Date of publication of application: 01.01.2025
(73) Proprietor: Devicor Medical Products, Inc., Cincinnati, OH 45241 (US)
(72) Inventor: RANDALL, Jack, A., Cincinnati, OH 45213 (US); HOUSEHOLDER, Garrett, A., Cincinnati, OH 45209 (US); GOODMAN, Devin, Cincinnati, OH 45238 (US)
(74) Representative: Maiwald GmbH
(86) International application number: PCT/US2023/018615
(87) International publication number: WO 2023/215090

(56) References cited:
- WO-A1-2021/217007
- US-A1- 2007 093 726
- US-A1- 2019 269 413
- US-A1- 2021 220 081

## Description

### BACKGROUND

A number of patients will have breast biopsies because of irregular mammograms and palpable abnormalities. Biopsies can include surgical excisional biopsies and stereotactic and ultrasound guided needle breast biopsies. In the case of image directed biopsy, the radiologist or other physician may take a small sample of the irregular tissue for laboratory analysis. If the biopsy proves to be malignant, additional surgery (e.g., a lumpectomy or a mastectomy) may be required. In the case of needle biopsies, the patient may return to the radiologist a day or more later, and the biopsy site (the site of the lesion) may need to be relocated in preparation for the surgery. An imaging system, such as ultrasound, magnetic resonance imaging (MRI) or x-ray may be used to locate the biopsy site. In order to assist the relocation of the biopsy site, a marker may be placed at the time of the biopsy.

The use of markers used after breast biopsies to mark the location where the biopsied tissue was removed is described in the following US Patents: US 6,083,524, "Polymerizable biodegradable polymers including carbonate or dioxanone linkages," issued July 4, 2000; US 6,162,241, "Hemostatic tissue sealants," issued December 4, 2000; US 6,270,464, "Biopsy localization method and device," issued August 7, 2001; US 6,356,782, "Subcutaneous cavity marking device and method," issued March 12, 2002; US 6,605,294, "Methods of using in situ hydration of hydrogel articles for sealing or augmentation of tissue or vessels," issued August 12, 2003; US 8,600,481, "Subcutaneous cavity marking device," issued December 3, 2013 and US 8,939,910, "Method for enhancing ultrasound visibility of hyperechoic materials", issued January 27, 2015.

Once a marker is placed at a biopsy site, the marker can later be relocated to identify the biopsy site in subsequent follow-up procedures. In some contexts, a placed marker may not completely correspond to the biopsy site when the marker is relocated. For instance, the marker may migrate to another nearby location during the intervening time between the biopsy procedure and subsequent follow up procedures. Migration of the biopsy site marker can cause difficulties when identifying the biopsy site during subsequent follow-up procedures. Accordingly, it may be desirable to incorporate features into a marker to maintain the marker in a fixed position over time.

Examples of markers with features configured to reduce migration are disclosed in US Pat. No. 10,842,591, entitled "Biopsy Marker with Anchoring Capabilities," issued on November 24, 2020; WO 2021/188481, entitled "Biopsy Site Markers with Non-Migration Features," published on September 23, 2021; and WO 2021/188479, entitled "Non-Migrating Biopsy Site Identifiers," published on September 23, 2021.
US2019/0269413 discloses a biopsy marker including a mono-filament which adopts a twisted or bent shape when deployed.

While several systems and methods have been made and used for marking a biopsy site, it is believed that no one prior to the inventor has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements. In the drawings some components or portions of components are shown in phantom as depicted by broken lines.
FIG. 1 depicts a perspective view of an example of a biopsy site marker;
FIG. 2 depicts a perspective view of a marker element of the marker of FIG. 1;
FIG. 3 depicts a front elevational view of the marker element of FIG. 2;
FIG. 4 depicts a side elevational view of the marker element of FIG. 2;
FIG. 5 depicts an exploded perspective view of the marker element of FIG. 2;
FIG. 6 depicts a perspective view of an alternative marker element that may be readily incorporated into the marker of FIG. 1;
FIG. 7 depicts a front elevational view of the marker element of FIG. 6;
FIG. 8 depicts a side elevational view of the marker element of FIG. 6;
FIG. 9 depicts a perspective view of another alternative marker element that may be readily incorporated into the marker of FIG. 1;
FIG. 10 depicts a front elevational view of the marker element of FIG. 9;
FIG. 11 depicts a perspective view of yet another alternative marker element that may be readily incorporated into the marker of FIG. 1;
FIG. 12 depicts a front elevational view of the marker element of FIG. 11;
FIG. 13 depicts a side elevational view of the marker element of FIG. 11.
FIG. 14 depicts a perspective view of still another alternative marker element that may be readily incorporated into the marker of FIG. 1;
FIG. 15 depicts a front elevational view of the marker element of FIG. 14;
FIG. 16 depicts a side elevational view of the marker element of FIG. 14;
FIGS. 17A, 17B, and 17C show exemplary aspects of placement of a biopsy site marker, in accordance with aspects of the present disclosure;
FIG. 18 depicts a perspective view of an exemplary marker delivery device;
FIG. 19 depicts a side cross-sectional view of the marker delivery device of FIG. 18; and
FIG. 20 depicts a cross-sectional view of a marker being deployed from the distal portion of the marker delivery device of FIG. 18 and through a lateral aperture in a biopsy needle to mark a biopsy site.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention, as long as they fall within the scope of the claims. Accordingly, the drawings and descriptions should be regarded as illustrative in nature and not restrictive.

It may be beneficial to be able to mark the location or margins of a lesion, whether temporarily or permanently, prior to or immediately after removing or sampling it. Marking prior to removal may help to ensure that the entire lesion is excised, if desired. Alternatively, if the lesion were inadvertently removed in its entirety, marking the biopsy site immediately after the procedure would enable reestablishment of its location for future identification.

Once a marker is positioned at a biopsy site, it may be desirable for the marker to remain visible under ultrasound. It may also be desirable to make the marker readily identifiable relative to other structural features of a patient. For instance, it may be desirable for the marker to be distinguishable under ultrasound visualization from microcalcifications to avoid inadvertently characterizing the marker as a microcalcification during subsequent ultrasonic examinations. Generally, microcalcifications are used in the field to identify suspicious lesions or masses. Thus, it is generally desirable for the ultrasound view to be distinguishable as a marker and not inadvertently identified as a new mass.

### I. Examples of Markers for Limited Migration and Enhanced Visualization

As described above, markers may be used in some contexts to identify the location of a biopsy site after a biopsy procedure is performed. Identification of the marker may be performed under a variety of imaging modalities such as ultrasound visualization, x-ray visualization, and/or etc. However, in the intervening time between the biopsy procedure and follow-up identification, some marker configurations may be susceptible to migration, leading to inaccuracies in subsequent identification of the biopsy site. Thus, it may be desirable in some circumstances for markers to include features configured to maintain the position of the marker within tissue.

When identifying the location of a marker, it may be desirable for the marker to have distinct characteristics under the imaging modality being used. Such distinct characteristics may make the marker more easily identifiable under the particular imaging modality being used by providing a contrast between natural tissue structures and the marker itself. Additionally, such distinct characteristics may be beneficial in contexts where multiple markers are used to marker different biopsy sites. Where multiple markers are used, it may be desirable to have one marker with a distinct visual profile relative to other markers to assist in distinguishing between biopsy sites.

### A. Examples of Markers with Helical Visualization Features

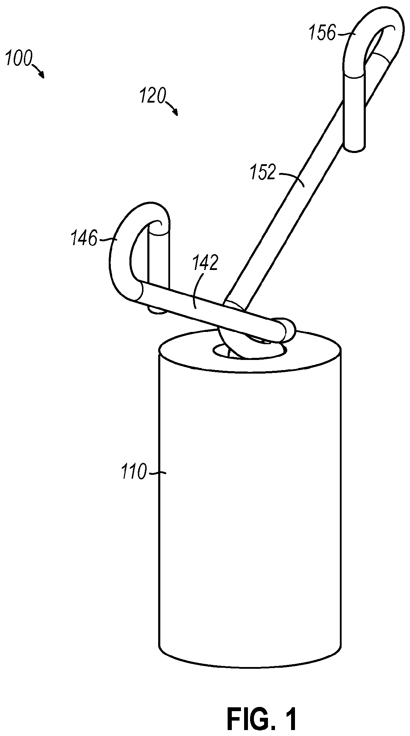

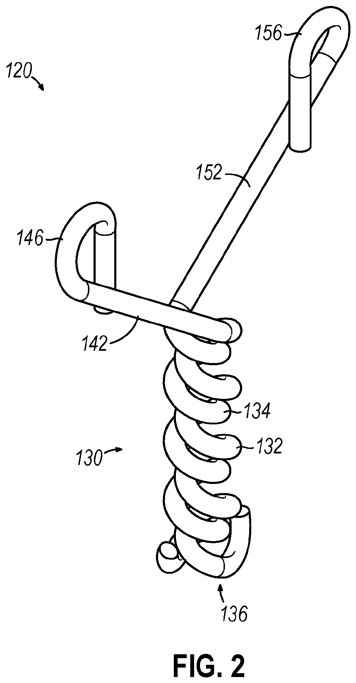

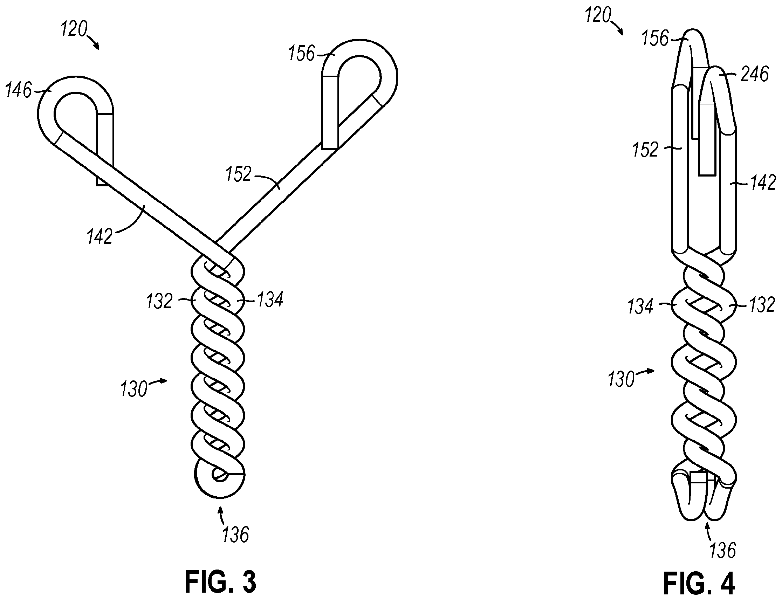

FIGS. 1 through 4 show an example of a marker (100) that is generally configured to anchor to tissue using anchors aligned along multiple planes to limit migration of marker (100) relative to an initial placement in tissue. Marker (100) is further generally configured with certain visualization features to provide a distinct appearance under various imaging modalities such as x-ray imaging as will be described in greater detail below. In the present version, marker (100) includes a carrier (110) and a marker element (120). Carrier (110) generally includes a bioabsorbable marker material (112). Thus, carrier (120) is generally configured for absorption into a patient after placement of marker (100) within a biopsy cavity, as will be described in greater detail below. In some versions, carrier (110) may include a plurality of microbubbles to enhance visualization of carrier (110) under ultrasound. As will be described in greater detail below, marker material (112) is generally bioabsorbable such that marker material (112) may be generally absorbed into the patient's tissue over time. Marker material (112) includes a hydrogel in the present version. Such hydrogel may be initially in a dehydrated state and absorb fluid to swell to a hydrated state prior to absorption into tissue. Although a hydrogel is used in the present version, it should be understood that in other versions, marker material (112) may include other suitable bioabsorbable materials.

Hydration of marker material (112) of carrier (110) by natural moisture of tissue surrounding may cause expansion of a polymer defining the hydrogel. This expansion may reduce the propensity of marker (100) to migrate within a patient. Such expansion may also serve to center marker (100) within a biopsy site as the expansion progresses. As the hydrogel expands, naturally present moisture from the surrounding tissue, the hydration enables increasing sound through transmission, appears more and more hypoechoic and is easy to visualize on follow up ultrasound studies.

Carrier (110) of the present example defines a generally cylindrical shape, although a variety of other shapes may be used. The particular shape of carrier (110) may be used in some versions to facilitate use with devices for deployment at a biopsy site. For instance, as will be described in greater detail below, in some versions marker (100) may be deployed at a biopsy site using a cylindrical tube or cannula. Thus, in such versions, carrier (110) being of a cylindrical shape may be desirable to promote ease of use with such cylindrical tubes or cannula. In other versions, carrier (110) may have one or more indentations, bulges, or an irregular shape to promote visualization of carrier (110) using an imaging modality such as ultrasound after deployment of marker (100) at a biopsy site. Of course, other configurations for carrier (110) may be used in other versions as will be apparent to those of ordinary skill in the art in view of the teachings herein.

Marker element (120) is generally configured as a permanent strcuture to permit identification of a biopsy site after absorption of carrier (110) into tissue. Thus, marker element (120) is generally non-bioabsorbable. Marker element (120) may include a radiopaque or echogenic marker, with at least a portion of marker element (120) being embedded with marker material (112) of carrier (110). For instance, marker element (120) may comprise metal, hard plastic, or other radiopaque or hyperechoic materials. Although marker element (120) in the present version is used with carrier (110), it should be understood that in some versions, carrier (110) may be omitted and marker (100) may include only marker element (120). In such versions, marker (100) may be referred to as a "bare" or "clip" style marker (100).

Marker element (120) includes a base portion (130) and a plurality of anchors (142, 152) (alternatively referred to as "outriggers") extending outwardly from base portion (130). In the present version, base portion (130) is generally disposed entirely within carrier (110). Base portion (130) is defined by one or more wires bent into a predetermined shape. Generally, the combination of the one or more wires forming base portion (130) are configured to provide a distinctive pattern to enhance visualization under x-ray and/or ultrasound visualization. Although base portion (130) is shown as having a particular orientation within carrier (110) (e.g., axially aligned) in the present version, it should be understood that base portion (130) may have a variety of alternative orientations in other versions.

Base portion (130) in the present version includes a first wire (132) and a second wire (134). Wires (132, 134) are twisted together to form a helical or spiral-shaped pattern. Due to the presence of two wires (132, 134) in the present version, this formation of wires (132, 134) may be characterized as a double-helix or double spiral pattern. Although two wires (132, 134) are used in the present version, it should be understood that in some versions, any other suitable number of wires (132, 134) may be used such as a single wire, three wires, four wires, or other suitable numbers.

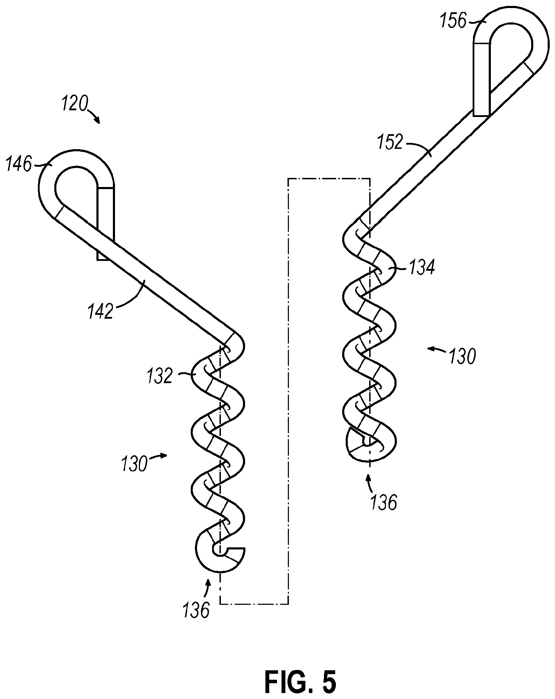

As best seen in FIG. 5, each wire (132, 134) has a substantially similar shape. Additionally, each wire (132, 134) is configured to intermesh with the other wire (134, 132) such that wires (132, 134) together form a double helix defining a generally cylindrical profile. The particular shape of each wire (132, 134) may be desirable in some versions to provide a distinct visual profile under x-ray visualization. The particular shape of each wire (132, 134) may also be desirable to provide a plurality of reflecting surfaces oriented at different angles to reflect ultrasonic energy and thereby provide enhanced ultrasonic visualization, particularly after absorption of carrier (110).

One end of each wire (132, 134) may be bent to provide an end portion (136). In the present version, end portion (136) is formed by each wire (132, 134) curving away from the other to form a generally rounded profile of end portion (136). The round profile shown in the present version may be desirable in some contexts to provide atraumatic properties with respect to tissue adjacent to marker (100).

Each wire (132, 134) terminates separately from the other wire (134, 132) at end portion (136). Thus, it should be understood that each wire (132, 134) is generally separate and discrete from the other wire (132, 134), with wires (132, 134) only being connected by the helical pattern formed in each wire (132, 134). In other words, each wire (132, 134) in the present version is a separate component. However, in other versions, wires (132, 134) may be connected into a single wire with two portions corresponding to first wire (132) and second wire (134). In other words, wires (132, 134) may be formed of a single continuum of material. In such versions, first wire (132) may curve to bend back upon itself to form second wire (134) at end portion (136).

Each anchor (142, 152) extends distally away from base portion (130) to protrude from a portion of carrier (110). The particular extension of each anchor (142, 152) includes a distal extension component and a lateral extension component. Thus, each anchor (142, 152) extends both distally and outwardly relative to base portion (130) and carrier (110). This combination of extension components is generally configured to promote engagement with tissue using each anchor (142, 152) even when carrier (110) is present prior to absorption of career (110).

Each anchor (142, 152) extends at an angle relative to a longitudinal axis defined by base portion (130). In the present version, the particular angle relative to the longitudinal axis may be about 55°. In other versions, the angle may be between about 50° and 60°. In yet other versions, the angle may be between about 45° to about 65°. Of course, in still other versions, various alternative angles may be used as will be apparent to those of ordinary skill in the art in view of the teachings herein.

Although each anchor (142, 152) is described herein as extending outwardly or laterally relative to a longitudinal axis defined by base portion (130), it should be understood that in some versions each anchor (142, 152) may be configured to move relative to the extension described herein. For instance, as will be described in greater detail below, marker (100) may be used in connection with a marker delivery device or other deployment apparatus having a cannula. In such uses, each anchor (142, 152) may move into a compressed configuration where each anchor (142, 152) is disposed within a circular footprint defined by carrier (110). In other words, each anchor (142, 152) may be compressed so that marker (100) may fit within a cannula with a diameter approximately corresponding to the diameter of carrier (110). Upon deployment from the cannula, each anchor (142, 152) may be released and move to the extended position described herein. Thus, the outward extension of each anchor (142, 152) described above may correspond to the natural or relaxed configuration of each anchor (142, 152) when being used to engage tissue. Alternatively, in some versions, the particular extension of each anchor (142, 152) may be responsive to hydration of carrier (110) such that the natural, relaxed, or fully extended configuration may not be reached until hydration of carrier (110).

Each anchor (142, 152) includes a respective anchor coil (146, 156) disposed on a distal end portion of each respective anchor (142, 152). Each anchor coil (146, 156) is generally configured to engage tissue to maintain marker (100) at a predetermined position at a biopsy site and to provide a distinct pattern to enhance visualization under x-ray and/or ultrasound visualization. The particular shape of each anchor coil (146, 156) includes one or more wire loops. In other words, each anchor coil (146, 156) is formed by a wire looped upon itself to provide an eyelet-shaped configuration. In this configuration, the one or more wire loops formed by each anchor coil (146, 156) may promote tissue in-growth to provide enhanced anchoring of marker (100). Although one or more circular loops are used in the present version for each anchor coil (146, 156), it should be understood that in other versions, different shapes may be used. Examples of suitable alternative shapes may include teardrop-shaped, square or rectangular-shaped, triangular-shaped, spiral-shaped, corkscrew-shaped, and/or etc. Alternatively, and as will be described in greater detail below, in some versions one or more anchor coils (146, 156) may be omitted and replaced with an end configuration with a non-coil shape. For instance, in some versions, one or more anchor coils (146, 156) may be replaced with a ridged, zig-zag, or M-shaped bent wire section.

Each anchor (142, 152) in the present version is integral with a corresponding first wire (132) and second wire (134), respectively. In other words, each anchor (142, 152) may be formed by a single piece of wire that also may form a portion of base portion (130). In other versions, one or more of anchors (142, 152) may be separately attached to base portion (130) by various coupling configurations. As described above with respect to base portion (130), first wire (132) and second wire (134) may be a single wire in some versions bent to form both first wire (132) and second wire (134). Thus, in such versions, each anchor (142, 152) may likewise be formed by a single continuous piece of wire bent to form first wire (132, second wire (134), and each anchor (142, 152).

Although not shown, it should be understood that each anchor (142, 152) may include one or more additional spring features or coils similar to anchor coils (146, 156), but oriented proximally of each anchor coil (146, 156). Such additional coils may be desirable to add a spring-based functionality to each anchor (142, 152) to permit compression of each anchor (142, 152) for use with a marker deployer and then expansion of each anchor (142, 152) upon deployment. Such additional coils may also be desirable to provide additional geometric features to facilitate tissue in-growth and/or other forms of tissue engagement.

In addition, or in the alternative, in some versions such a spring-based functionality may be facilitated by materials used for each anchor (142, 152). For instance, in some versions, each anchor (142, 152) may include a shape memory alloy processed to have a pre-deployment shape and a post-deployment shape. Each anchor (142, 152) may then be configured to transition from the pre-deployment shape to the post-deployment shape based on the temperature of tissue and/or other associated temperatures.

Each anchor (142, 152) in the present version is shown as being substantially similar in size, shape, and configuration. However, also in the present version, one anchor (152) may be distally offset from another anchor (142). In other words, anchor (152) may include a distal projection prior to extending distally and laterally, while such a distal projection may be omitted from anchor (142). The net result of this configuration is one anchor (152) being oriented a greater distance distally relative to another anchor (142) and base portion (130). Although the present version includes a distal offset with respect to anchor (152), it should be understood that in some versions such a distal offset may be omitted or alternatively used with both anchors (142, 152).

Although each anchor (142, 152) in the present version is generally substantially similar in size, shape, and configuration, it should be understood that in other versions, each anchor (142, 152) may define a different configuration relative to the other (152, 142). For instance, in some versions, one anchor (142, 152) may be longer relative to the other anchor (152, 142). In addition, or in the alternative, one anchor (142, 152) may include anchor coil (146, 156) as described herein, while another anchor (152, 142) may omit structures similar to anchor coil (146, 156) or use a different strcuture such as a ridged configuration described in greater detail below. In addition, or in the alternative, one anchor (142, 152) may include additional features such as a spring feature, compound angle extension, and/or etc., while another anchor (152, 142) may exclude such features. Of course, various alternative configurations for each anchor (142, 152), either individually or together, will be apparent to those of ordinary skill in the art in view of the teachings herein.

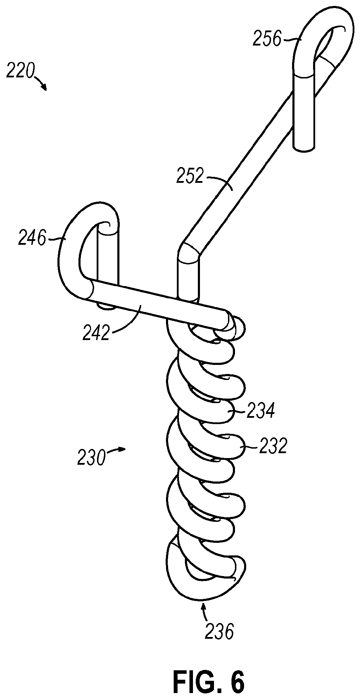

FIGS. 6 through 8 show an example of an alternative marker element (220) that may be incorporated into marker (100) described above in addition to or in lieu of marker element (120). Marker element (220) is generally substantially similar to marker element (120) described above. For instance, like with marker element (120), marker element (220) of the present version is generally configured as a non-bioabsorbable marker that may be radiopaque and/or echogenic in nature. Marker element (220) may similarly be used in connection with carrier (110) as described above or alternatively used without carrier (110) to form a bare or clip-style marker (100).

As with marker element (120) described above, marker element (220) of the present version includes a base portion (230) and a plurality of anchors (242, 252) (alternatively referred to as "outriggers"). As with base portion (130) described above, base portion (230) of the present version may be disposed within carrier (110), while anchors (242, 252) may project distally from carrier (110). Base portion (230) may be configured similarly to base portion (130) in that base portion (230) may include one or more wires bent into a predetermined shape to provide a distinctive pattern that may enhance visualization under x-ray and/or ultrasound visualization. The particular shape used in the present version is a helical or spiral-shaped pattern similar to the pattern described above with respect to base portion (130).

Each anchor (242, 252) in the present version is substantially similar to anchors (142, 152) described above. For instance, as with anchors (142, 152) described above, each anchor (242, 252) in the present version may extend distally from base portion (230) to protrude from at least a portion of carrier (110). Similarly, the extension of each anchor (242, 252) may include a distal component and an outward component to define an extension angle relative to a longitudinal axis of base portion of about 55° in some versions, from 50° to 60° in some versions, and from about 45° to 65° degrees in other versions. As with anchors (142, 152) described above, anchors (242, 252) of the present version may similarly be configured to move to the outwardly extending configuration described herein to engage tissue from a compressed configuration for use with a marker delivery device.

Each anchor (242, 252) also includes a respective anchor coil (246, 256) disposed on a distal end of each respective anchor (242, 252) similar to anchor coils (146, 156) described above. As similarly described above, each anchor coil (246, 256) is generally configured to engage tissue to maintain marker (100) at a predetermined postion at a biopsy site and to provide a distinct pattern to enhance visualization under x-ray and/or ultrasound visualization. The particular shape of each anchor coil (246, 256) is substantially similar to anchor coils (146, 156) described above in that each anchor coil (246, 256) includes one or more loops of wire to form an eyelet-shaped configuration. As similarly described above, in other versions, each anchor coil (246, 256) may define other suitable shapes such as teardrop-shaped, square or rectangular-shaped, triangular-shaped, spiral-shaped, corkscrew-shaped, and/or etc. As also similarly described above, in some versions, one or more anchor coils (246, 256) may be omitted and replaced with an end configuration with a non-coil shape such as ridged, zig-zag, or M-shaped bent wire sections.

Unlike marker element (120) described above, marker element (220) of the present version includes single-wire configuration. For instance, base portion (230) of the present version defines a first wire portion (232) and a second wire portion (234). Both first wire portion (232) and second wire portion (234) are substantially similar to first wire (132) and second wire (134) described above in terms of together defining a double-helix shape by two separate wire portions being bent into a helical or spiral pattern. However, instead of wire portions (232, 234) terminating at an end portion (236) to define separate wire elements, wire portions (232, 234) are integral with each other such that the entirety of marker element (220) is formed of a single unitary wire bent into the particular shape shown.

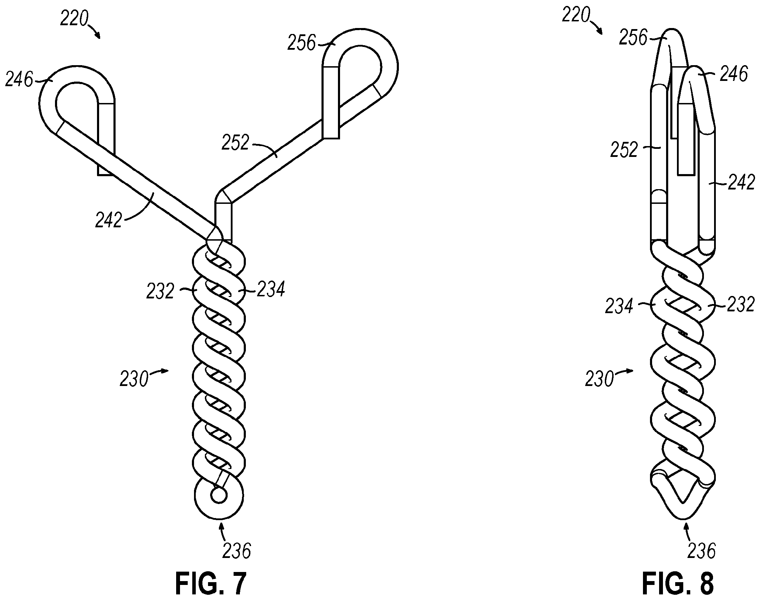

As best seen in FIGS. 7 and 8, end portion (236) defines a generally curved circular or oval-shaped profile at the proximal end of base portion (230). In particular, first wire portion (232) may bend around the distal end of base portion (230) to intersect with second wire portion (234). In other words, end portion (236) forms a coil at the proximal end of base portion (230). In some versions, end portion (236) may include multiple coil loops such as two, three, or more. Although end portion (236) defines a curved circular or oval-shaped profile in the present version, it should be understood that in other versions, various alternative shapes may be used. Suitable alternative shapes for end portion (236) may include rectangular or square-shaped, triangular-shaped, hexagonal-shaped, diamond-shaped, trapezoid-shaped, and/or etc.

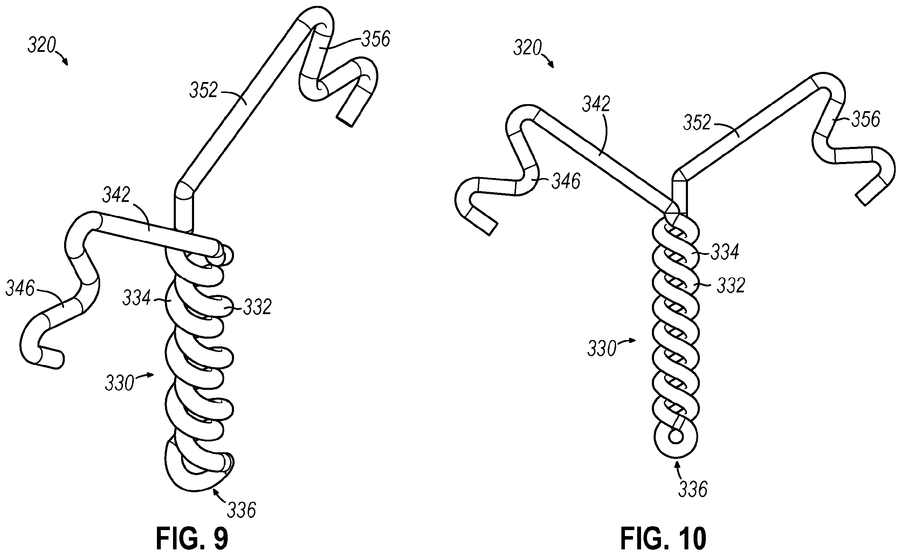

FIGS. 9 and 10 show an example of an alternative marker element (320) that may be incorporated into marker (100) described above in addition to or in lieu of marker elements (120, 220). Marker element (320) is generally substantially similar to marker elements (220) described above. For instance, like with marker element (220), marker element (320) of the present version is generally configured as a non-bioabsorbable marker that may be radiopaque and/or echogenic in nature. Marker element (320) may similarly be used in connection with carrier (110) as described above or alternatively used without carrier (110) to form a bare or clip-style marker (100).

As with marker element (220) described above, marker element (320) of the present version includes a base portion (330) and a plurality of anchors (342, 352) (alternatively referred to as "outriggers"). As with base portion (230) described above, base portion (330) of the present version may be disposed within carrier (110), while anchors (342, 352) may project distally from carrier (110). Base portion (330) may be configured similarly to base portion (230) in that base portion (330) may include one or more wires bent into a predetermined shape to provide a distinctive pattern that may enhance visualization under x-ray and/or ultrasound visualization. The particular shape used in the present version is a helical or spiral-shaped pattern similar to the pattern described above with respect to base portion (230).

Marker element (320) of the present version includes a single-wire configuration similar to the single-wire configuration described above with respect to marker element (220). As similarly described above, base portion (330) defines a first wire portion (332) and a second wire portion (334) that intersect at an end portion (336) defining a curved or other alternative shape. As also similarly described above, both first wire portion (332) and second wire portion (334) may be bent into a twisted double-helix or spiral shape that defines the shape of base portion (330). Although a single-wire configuration is used in the present version to define the shape of base portion (330), it should be understood that in other versions, a multi-wire configuration may be used similar to the multi-wire configuration described herein with respect to base portion (130).

Each anchor (342, 352) in the present version is similar to anchors (242, 252) described above. For instance, as with anchors (242, 252) described above, each anchor (342, 352) in the present version may extend distally from base portion (330) to protrude from at least a portion of carrier (110). Similarly, the extension of each anchor (342, 352) may include a distal component and an outward component to define an extension angle relative to a longitudinal axis of base portion of about 55° in some versions, from 50° to 60° in some versions, and from about 45° to 65° degrees in other versions. As with anchors (242, 252) described above, anchors (342, 352) of the present version may similarly be configured to move to the outwardly extending configuration described herein to engage tissue from a compressed configuration for use with a marker delivery device.

Unlike anchors (242, 252) described above, each anchor (342, 352) omits structures similar to anchor coils (246, 256) described above. Instead, each anchor (342, 352) includes an anchor member (346, 356) disposed on a distal end of each respective anchor (342, 352). Each anchor member (346, 356) is generally configured to engage tissue to maintain marker (100) at a predetermined postion at a biopsy site and to provide a distinct pattern to enhance visualization under x-ray and/or ultrasound visualization. The shape of each anchor member (346, 356) is of an irregular projection rather than the coil-shaped described above with respect to anchor coils (246, 256). The particular irregular shape used in the present version of each anchor member (346, 356) is M-shaped or ridged. In some versions, this shape may be desirable to provide increased engagement with tissue and/or a more distinct profile under x-ray visualization. Although an M-shape is used in the present version, it should be understood that various alternative shapes defining a series of ridges may be used in other versions. In other versions, one or more of anchor members (346, 356) may alternatively include one or more loops of wire to form an eyelet-shaped configuration similar to anchor coils (246, 256) described above.

### B. Example of Marker Element with Crossing Visualization Features

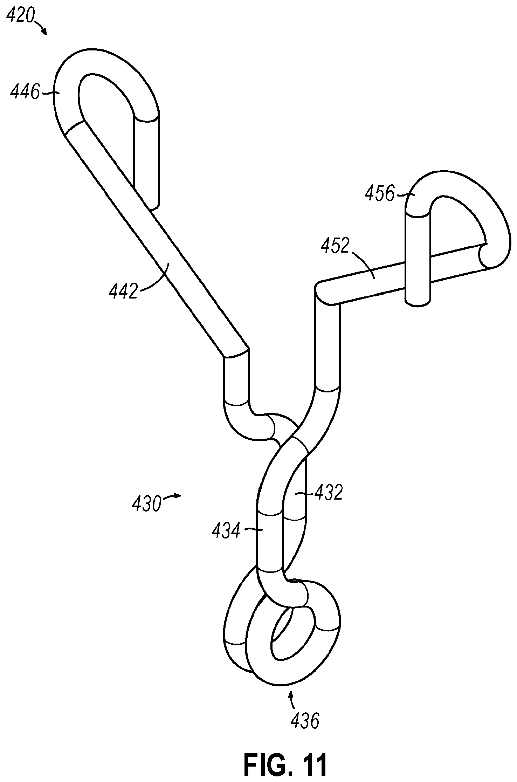

FIGS. 11 through 13 show an example of an alternative marker element (420) that may be incorporated into marker (100) described above in addition to or in lieu of marker elements (120, 220, 320). Marker element (320) is generally similar to marker elements (120, 220, 320) described above. For instance, like with marker elements (120, 220, 320), marker element (420) of the present version is generally configured as a non-bioabsorbable marker that may be radiopaque and/or echogenic in nature. Marker element (420) may similarly be used in connection with carrier (110) as described above or alternatively used without carrier (110) to form a bare or clip-style marker (100).

As with marker elements (120, 220, 320) described above, marker element (420) of the present version includes a base portion (430) and a plurality of anchors (442, 452) (alternatively referred to as "outriggers"). As with base portions (130, 230, 330) described above, base portion (430) of the present version may be disposed within carrier (110), while anchors (442, 452) may project distally from carrier (110).

Each anchor (442, 452) in the present version is similar to anchors (142, 152, 242, 252) described above. For instance, as with anchors (142, 152, 242, 252) described above, each anchor (442, 452) in the present version may extend distally from base portion (430) to protrude from at least a portion of carrier (110). Similarly, the extension of each anchor (442, 452) may include a distal component and an outward component to define an extension angle relative to a longitudinal axis of base portion of about 55° in some versions, from 50° to 60° in some versions, and from about 45° to 65° degrees in other versions. As with anchors (142, 152, 242, 252) described above, anchors (442, 452) of the present version may similarly be configured to move to the outwardly extending configuration described herein to engage tissue from a compressed configuration for use with a marker delivery device.

Each anchor (442, 452) also includes a respective anchor coil (446, 456) disposed on a distal end of each respective anchor (442, 452) similar to anchor coils (146, 156, 246, 256) described above. As similarly described above, each anchor coil (446, 456) is generally configured to engage tissue to maintain marker (100) at a predetermined postion at a biopsy site and to provide a distinct pattern to enhance visualization under x-ray and/or ultrasound visualization. The particular shape of each anchor coil (446, 456) is substantially similar to anchor coils (146, 156, 246, 256) described above in that each anchor coil (446, 456) includes one or more loops of wire to form an eyelet-shaped configuration. As similarly described above, in other versions, each anchor coil (446, 456) may define other suitable shapes such as teardrop-shaped, square or rectangular-shaped, triangular-shaped, spiral-shaped, corkscrew-shaped, and/or etc. As also similarly described above, in some versions, one or more anchor coils (446, 456) may be omitted and replaced with an end configuration with a non-coil shape such as ridged, zig-zag, or M-shaped bent wire sections similar to the configuration described above with respect to anchor members (346, 356) of marker element (320).

Marker element (420) of the present version includes a single-wire configuration similar to the single-wire configuration described above with respect to marker elements (220, 320). As similarly described above, base portion (430) defines a first wire portion (432) and a second wire portion (434) that intersect at an end portion (436) defining a curved or other alternative shape. As also similarly described above, both first wire portion (432) and second wire portion (434) may be bent into a predetermined shape defining the shape of base portion (430). Although a single-wire configuration is used in the present version to define the shape of base portion (430), it should be understood that in other versions, a multi-wire configuration may be used similar to the multi-wire configuration described herein with respect to base portion (130).

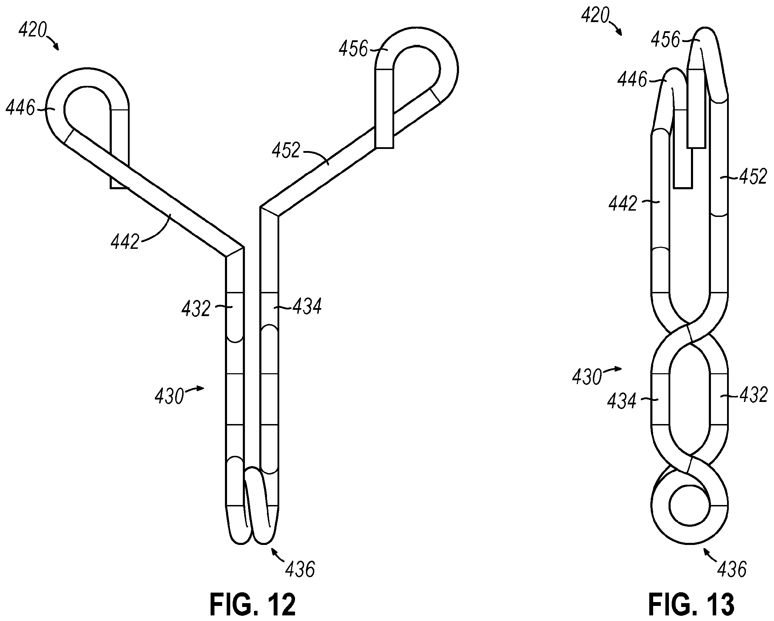

Although end portion (436) is similar to end portions (236, 336) described above in that end portion (436) defines a curved shape, end portion (436) differs from end portions (236, 336) described above in that end portion (436) includes a plurality of loops or a plurality of curvatures. In particular, as best seen in FIG. 12, wire portions (432, 434) bend toward each other to define multiple coils at end portion (436). Such a multiple coil configuration may be desirable in some versions to provide additional structural stability to marker element (420). Although two coils are defined in the present version, in other versions other suitable numbers of coils may be used such as two or more, or one similar to the configuration described above with respect to end portions (236, 336).

Unlike base portions (130, 230, 330) described above, base portion (430) defines a different shape relative to the spiral-shaped configuration described above. In particular, each of first wire portion (432) and second wire portion (434) in the present version may be bent to define a crossing pattern. Such a crossing pattern may be desirable in some versions to define a distinct appearance of base portion (430) under x-ray visualization relative to the shapes described above with respect to base portions (130, 230, 330). The crossing pattern defined in the present example specifically includes each wire portion (432, 434) first diverging outwardly in a opposite direction relative to another wire portion (432, 434). Wire portions (432, 434) then extend parallel to each other for a predetermined length before converging inwardly to cross one another. In this configuration, wire portions (432, 434) together define an approximately rounded rectangle configuration when viewed from the side as shown in FIG. 13.

### C. Example of Marker Element with Linear Visualization Features

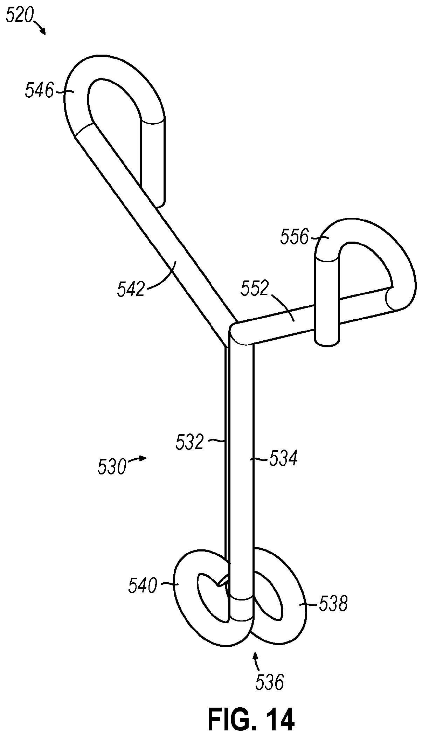

FIGS. 14 through 16 show an example of an alternative marker element (520) that may be incorporated into marker (100) described above in addition to or in lieu of marker elements (120, 220, 320, 420). Marker element (420) is generally similar to marker elements (120, 220, 320, 420) described above. For instance, like with marker elements (120, 220, 320, 420), marker element (520) of the present version is generally configured as a non-bioabsorbable marker that may be radiopaque and/or echogenic in nature. Marker element (520) may similarly be used in connection with carrier (110) as described above or alternatively used without carrier (110) to form a bare or clip-style marker (100).

As with marker elements (120, 220, 320, 420) described above, marker element (520) of the present version includes a base portion (530) and a plurality of anchors (542, 552) (alternatively referred to as "outriggers"). As with base portions (130, 230, 330, 430) described above, base portion (530) of the present version may be disposed within carrier (110), while anchors (542, 552) may project distally from carrier (110).

Each anchor (542, 552) in the present version is similar to anchors (142, 152, 242, 252, 442, 452) described above. For instance, as with anchors (142, 152, 242, 252, 442, 452) described above, each anchor (542, 552) in the present version may extend distally from base portion (530) to protrude from at least a portion of carrier (110). Similarly, the extension of each anchor (542, 552) may include a distal component and an outward component to define an extension angle relative to a longitudinal axis of base portion of about 55° in some versions, from 50° to 60° in some versions, and from about 45° to 65° degrees in other versions. As with anchors (142, 152, 242, 252, 442, 452) described above, anchors (542, 552) of the present version may similarly be configured to move to the outwardly extending configuration described herein to engage tissue from a compressed configuration for use with a marker delivery device.

Each anchor (542, 552) also includes a respective anchor coil (546, 556) disposed on a distal end of each respective anchor (542, 552) similar to anchor coils (146, 156, 246, 256, 446, 456) described above. As similarly described above, each anchor coil (546, 556) is generally configured to engage tissue to maintain marker (100) at a predetermined postion at a biopsy site and to provide a distinct pattern to enhance visualization under x-ray and/or ultrasound visualization. The particular shape of each anchor coil (546, 556) is substantially similar to anchor coils (146, 156, 246, 256, 446, 456) described above in that each anchor coil (546, 556) includes one or more loops of wire to form an eyelet-shaped configuration. As similarly described above, in other versions, each anchor coil (546, 556) may define other suitable shapes such as teardrop-shaped, square or rectangular-shaped, triangular-shaped, spiral-shaped, corkscrew-shaped, and/or etc. As also similarly described above, in some versions, one or more anchor coils (546, 556) may be omitted and replaced with an end configuration with a non-coil shape such as ridged, zig-zag, or M-shaped bent wire sections similar to the configuration described above with respect to anchor members (346, 356) of marker element (320).

Marker element (520) of the present version includes a multi-wire configuration similar to the multi-wire configuration described above with respect to marker element (120). As similarly described above, base portion (530) defines a first wire (532) and a second wire (534) that extend to an end portion (536), but remain separate at end potion (536). As also similarly described above, both first wire (532) and second wire (534) may be bent into a predetermined shape defining the shape of base portion (530). Although a multi-wire configuration is used in the present version to define the shape of base portion (530), it should be understood that in other versions, a single-wire configuration may be used similar to the single-wire configuration described herein with respect to base portions (230, 330, 430).

End portion (536) is similar to end portion (136) described above in that end portion (536) includes a curvature with the proximal end of each of first wire (532) and second wire (534) bent in different or opposite directions. However, unlike end portion (136) described above, each of first wire (532) and second wire (534) are bent to form a first end coil (538) and second end coil (540), respectively. Each end coil (538, 540) defines a loop of wire or coil that defines the curved shape of end portion (536). Each end coil (538, 540) is also oriented about parallel planes such that end coils (538, 540) may be arranged in a side-by-side, yet offset configuration. Although each end coil (538, 540) in the present version includes a single coil, it should be understood that in some versions first end coil (538), second end coil (540), or both may include two or more coils.

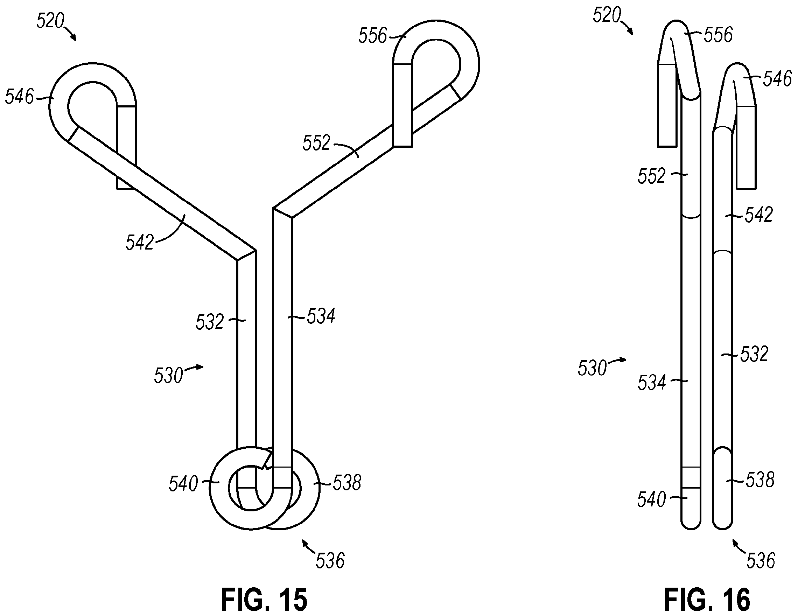

Unlike base portions (130, 230, 330) described above, base portion (530) defines a different shape relative to the spiral-shaped configuration described above. In particular, each of first wire (532) and second wire (534) in the present version may be bent to define a generally linear pattern. In other words, first wire (532) and second wire (534) may together define a profile having two parallel lines as seen in FIGS. 15 and 16. Additionally, first wire (532) and second wire (534) may be offset relative to each other in one or more lateral directions. As a result, wires (532, 534) may be entirely separate from each other or not touching. In this configuration, the particular relative position of first wire (532) and second wire (534) may be held in position using carrier (110) as opposed to a physical connection between wires (532, 534). In other words, carrier (110) may function as a fastener in some versions to hold wires (532, 534) in a predetermined position. Such a configuration may be desirable in some versions to define a distinct appearance of base portion (530) under x-ray visualization relative to the shapes described above with respect to base portions (130, 230, 330, 430).

### II. Example Uses of Markers for Marking a Biopsy Site

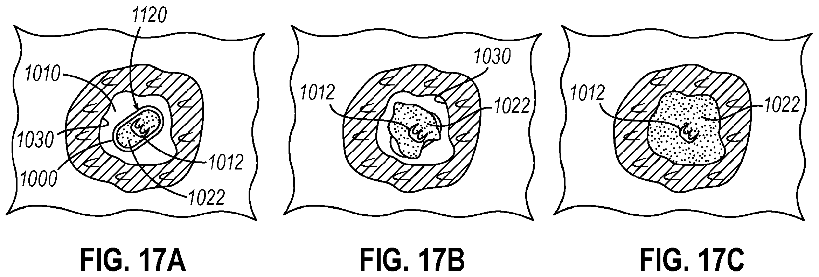

FIGS. 17A through 17C show aspects of a procedure for percutaneously marking a biopsy cavity (1010) having surrounding tissue (1030). For instance, as seen in FIG. 17A, a marker (1000) may be initially placed in the biopsy cavity (10) to facilitate relocation of the biopsy site. Although marker (1000) shown herein includes specific characteristics, it should be understood that in some versions marker (100) described above may also be used in connection with biopsy cavity (1010) and surrounding tissue (1030). As with marker (100) described above, marker (1000) may comprise a carrier (1200) similar to carrier (120) and a marker element (1012) similar to marker elements (120, 220, 320, 420, 520). As with carrier (120) described above, carrier (1120) generally includes a bioabsorbable marker material (1022). Thus, carrier (1120) is generally configured for absorption into a patient after placement of marker (1000) within the biopsy cavity (1010). In some versions, carrier (1120) can include a plurality of microbubbles to enhance visualization of carrier (1120) under ultrasound. As will be described in greater detail below, marker material (1022) is generally bioabsorbable such that marker material (1022) may be generally absorbed into the patient's tissue over time. In the present version, marker material (1022) comprises a hydrogel that is initially in a dehydrated state. Although a hydrogel is used in the present version, it should be understood that in other versions marker material (1022) may comprise other known bioabsorbable materials.

In the present version, marker (1000) further includes a marker element (1012) that is generally not bioabsorbable. Although not shown, it should be understood that in some versions, marker element (1012) may include one or more aspects of marker elements (120, 220, 320, 420, 520) described above such as aspects of base portions (130, 230, 330, 430, 530), and/or anchors (142, 152, 242, 252, 342, 352, 442, 452, 542, 552). As with marker elements (120, 220, 320, 420, 520) described above, marker element (1012) may comprise a radiopaque or echogenic marker embedded within the bioabsorbable marker material (1022) of carrier (1120). For instance, marker element (1012) may comprise metal, hard plastic, or other radiopaque or hyperechoic materials known to those of ordinary skill in the art in view of the teachings herein. In other versions, marker (1000) may be formed without a marker element (1012). In still other versions, marker (1000) may be formed with only marker element (1012) such that carrier (1120) is omitted and marker element (1012) is in a "bare" form. In other words, in some versions marker (1000) is formed of only marker element (1012) as a bare clip.

Marker material (1022) is generally expandable once disposed within a patient at biopsy cavity (1010) of a biopsy site. As shown in FIGS. 17B and 17C, the initially dehydrated marker material (1022) may absorb fluid from the surrounding tissue (1030) into which it is inserted. In response to this absorption of fluid, maker material (1022) may swell, thereby permitting carrier (1120) to fill biopsy cavity (1010) formed at a biopsy site by removal of tissue samples during a biopsy procedure. Biodegradable materials may be particularly suitable in applications where it is desired that natural tissue growth be permitted to completely or partially replace the implanted material over time. Accordingly, biocompatibility is ensured and the natural mechanical parameters of the tissue are substantially restored to those of the pre-damaged condition.

Marker (1000) may be inserted into the body either surgically via an opening in the body cavity (1030), or through a minimally invasive procedure using such devices as a catheter, introducer or similar type insertion device. Marker (1000) may be delivered immediately after removal of the tissue specimen using the same device used to remove the tissue specimen itself. Follow-up noninvasive detection techniques, such as x-ray mammography or ultrasound may then be used by the physician to identify, locate, and monitor the biopsy cavity site over a period of time via marker (1000).

Marker (1000) of the present version is large enough to be readily visible to a clinician under x-ray or ultrasonic viewing, for example; yet small enough to be able to be percutaneously deployed into the biopsy cavity and to not cause any difficulties with the patient. Although versions are described in connection with treatment and diagnosis of breast tissue, aspects presented herein may be used for markers in any internal, tissue, e.g., in breast tissue, lung tissue, prostate tissue, lymph gland tissue, etc.

The hydration of the marker material (1022) of carrier (1120) by the natural moisture of the tissue surrounding it causes expansion of the polymer and thus minimizes the risk of migration. The growing hydrogel-based marker material (1022) centers marker (1000) in the biopsy cavity as it grows. As the hydrogel expands, naturally present moisture from the surrounding tissue, the hydration enables increasing sound through transmission, appears more and more hypoechoic and is easy to visualize on follow up ultrasound studies.

The hydrated hydrogel marker material (1022) of carrier (1120) may also be used to frame permanent marker (1012). The hypoechoic nature of the hydrated marker material (1022) enables ultrasound visibility of the permanent marker (102) within the hydrogel hydrated marker material (1022) because the permanent marker (1012) is outlined as a specular reflector within a hypoechoic hydrated marker having a waterlike nonreflective substrate.

### III. Exemplary Marker Delivery Device

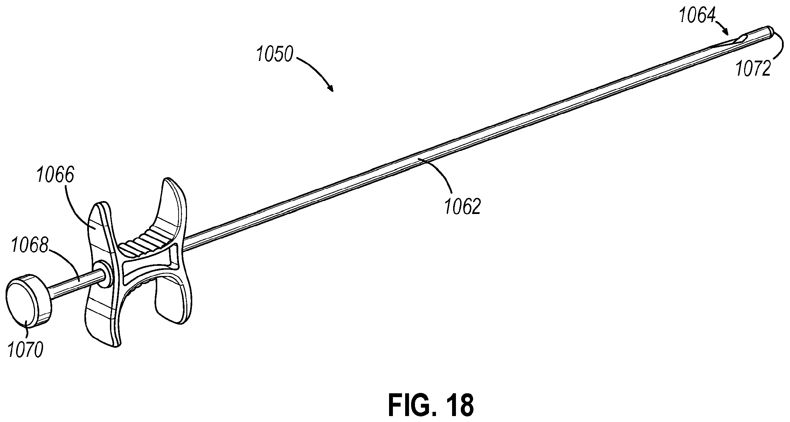

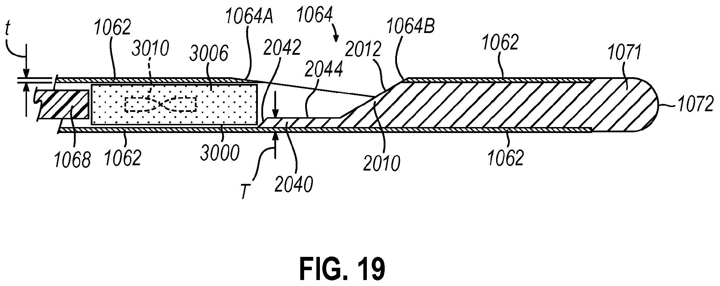

In some versions it may be desirable to deploy any one or more of markers (100, 1000) described above within biopsy cavity (1030) using certain marker delivery devices. For instance, FIGS. 18 and 19 show an exemplary marker delivery device (1050) which includes an elongate outer cannula (1062) having a marker exit, such as side opening (1064) formed adjacent to, but spaced proximally from, the distal end of the cannula (1062).

Although marker delivery device (1050) is shown herein as being used with a marker (3000), it should be understood that in other uses, marker delivery device (1050) may be readily used with any one or more of markers (100, 1000) described herein. Additionally, in some uses, marker delivery device (1050) may come equipped with a marker kit including several versions of marker (100) and an operator may select one or more of such version of marker (100) for use with marker delivery device (1050). For instance, in use with such a kit, several versions of marker (100) having one or more of marker elements (120, 220, 320, 420, 520) described above may be included in the kit. An operator may then choose a particular version of marker (100) or combination of different versions of marker (100) for use with maker delivery device (1050). Such a use may be desirable to provide an operator preferred distinct appearance of marker (100) for later identification of a particular biopsy site. Such operator preference may be particularly desirable in circumstances where multiple biopsy sites are present in a single patient either from a single procedure or multiple procedures over time.

A grip (1066) can be provided at the proximal end of cannula (1062). A push rod (1068) can be provided, with push rod (1068) extending coaxially in cannula (1062) such that push rod (1068) is configured to translate within cannula (1062) to displace one or more markers through side opening (1064) *(see* FIG. 19). Rod (1068) may have sufficient rigidity in compression to push a marker from an internal lumen of cannula (1062) out through opening (1064), yet be relatively flexible in bending. A plunger (1070) is coupled at the proximal end of rod (1068) for forcing rod (1068) distally in cannula (1062) to deploy a marker out of cannula (1062).

A user may grasp grip (1066) with two fingers, and may push on plunger (1070) using the thumb on the same hand, so that marker delivery device (1060) is operated by a user's single hand. A spring (not shown) or other feature may be provided about rod (1068) to bias rod (1068) proximally relative to grip (1066) and cannula (1062).

FIG. 19 shows a cross-sectional view of a distal portion of the marker delivery device (1060). As can be seen, a biopsy marker (3000) similar to markers (100, 1000) described above is disposed within internal lumen (1065) of cannula (1062). In the present version, marker (3000) includes a biodegradable or otherwise resorbable marker material (3006), such as a generally cylindrically shaped body of collagen, hydrogel, or etc., and a metallic, generally radiopaque permanent marker or marker element (3010) (shown in phantom) disposed within or otherwise carried by marker material (3006).

Cannula (1062) may be formed of any suitable metallic or non-metallic material. In some versions, cannula (1062) is formed of a thin-walled hollow tube formed of a suitable medical grade plastic or polymer. One suitable material is a thermoplastic elastomer, such as Polyether block amide (PEBA), such as is known under the tradename PEBAX. Cannula (1062) may be formed of PEBAX, and may be substantially transparent to visible light and X-ray.

Side opening (1064) may be formed by cutting away a portion of the wall of cannula (1062). Side opening (1064) communicates with an internal lumen of cannula (1062). Side opening (1064) may extend axially (in a direction parallel to the axis of lumen (165)) from a proximal opening end (1064A) to a distal opening end (1064B), as illustrated in FIG. 19.

In the present version, distal tip (1072) extends from the distal end of cannula (1062) and is rounded as shown in FIG. 19. The distal end of cannula (1062) is closed by a unitary endpiece (1071), with a portion of endpiece (1071) extending into a portion of cannula (1062). Endpiece (1071) may be a molded or cast component. Endpiece (1071) comprises a tip (1072), a ramp (2010) having a ramp surface (2012), and a marker engaging element (2040). Ramp surface (2012) aids in directing marker (3000) from internal lumen of cannula (1062) through side opening (1064). Marker engaging element (2040) helps to retain marker (3000) in cannula (1062) until the user intends to deploy marker (3000).

Marker engaging element (2040) is disposed within cannula (1062), and at least a portion of marker engaging element (2040) is disposed distally of proximal end (1064A) of side opening (1064). Marker engaging element (2040) extends along a portion of the floor of cannula (1062) under opening (1064) such that marker engaging element (2040) is positioned to reinforce the portion of cannula (1062) in which opening (1064) is formed. For instance, by positioning marker engaging element (2040) underneath opening (1064), as shown in FIG. 19, element (2040) helps to stiffen cannula (1062) in the region where wall of cannula (1062) is cut to form opening (1064). As shown in FIG. 19, marker engaging element (2040) extends from the proximal most portion of ramp surface (2012), and does not extend proximally of side opening (1064), though in other embodiments, a portion of element (2040) may extend proximally of opening (1064).

As shown in FIG. 19, marker engaging element (2040) is in the form of a step having a generally uniform thickness (T) along element's (2040) axial length, except that element (2040) has a tapered proximal end (2042). Tapered proximal end (2042) forms an included angle with the longitudinal axis of cannula (1062) (included angle with a horizontal line in FIG. 19) of about 45 degrees, while ramp surface (2012) forms an included angle with the longitudinal axis of about 30 degrees. Of course, any number of other suitable angles may be used.

As shown in FIG. 19, an upwardly facing surface (2044) (surface facing opening (1064)) of marker engaging element (2040) extends distally to contact ramp surface (2012), so that there is not a space or gap between surface (2044) and ramp surface (2012). Such an arrangement is advantageous to reduce the possibility that marker (3000), upon moving past marker engaging element (2040), may become lodged between marker engagement element (2040) and ramp (2012). In some versions, marker engaging element (2040), ramp (2010), and/or tip (1072) are formed of, or include, a material that is relatively more radiopaque than the wall of cannula (1062). For instance, where element (2040), ramp (2010), and tip (1072) are formed as an integral endpiece (1071), endpiece (1071) may include a radiopaque additive, such as barium sulfate. For instance, endpiece (1071) may be a component molded of PEBAX, with about 20 percent by weight barium sulfate added to the molten PEBAX mold composition. The relatively more radiopaque marker engaging element (2040), ramp (2010), and tip (1072) may be useful in distinguishing the position of those components using radiographic imaging. Also, where ramp (2010) and/or step of engaging element (2040) are positioned in association with opening (1064), the addition of a radiopaque material can help identify the position of opening (1064), and the position of marker (3000) relative to opening (1064) before, during, or after deployment of marker (3000).

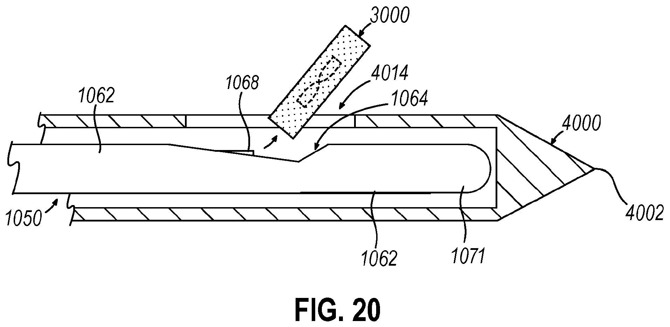

Referring to FIG. 20, marker delivery device (1060) is used to deploy a marker (3000) to mark a biopsy location within a patient. In FIG. 20, a cannular biopsy needle (4000) is shown having a closed distal end with piercing tip (4002) and a lateral tissue receiving aperture (4014). Marker delivery device (1060) is introduced to a biopsy site through biopsy needle (4000), which may be the same needle (4000) used to collect a tissue sample from the biopsy site. Biopsy needle (4000) may be of the type used with single insertion, multiple sample vacuum assisted biopsy devices. Several such biopsy devices are disclosed in the various patents and patent applications that have been referred to, though other biopsy devices may be used.

FIG. 20 shows the distal end of marker delivery device (1060) disposed within needle (4000). Needle (4000) may be positioned in tissue, and a biopsy sample may be obtained through lateral aperture (4014), thereby providing a biopsy cavity adjacent lateral aperture (4014). Then, after the tissue sample has been obtained and transferred proximally through needle (4000), and without removing needle (4000) from the patient's tissue, marker delivery device (1060) is inserted into a proximal opening in needle (4000). In FIG. 20, needle (4000) and marker delivery device (1060) are positioned such that opening (1064) of cannula (1062) and lateral aperture (4014) of needle (4000) are substantially aligned axially and circumferentially. Then, with marker delivery device (1060) and needle (4000) so positioned at the biopsy site, push rod (1068) is advanced to deploy marker (3000) up ramp surface (2012), through opening (1064), and then through lateral aperture (4014), into the biopsy cavity.

## Claims

1. A biopsy site marker, comprising:
a carrier; and
a marker element (420) including a base portion (430), a first anchor (442), and a second anchor (452), the base portion (430) being at least partially disposed within the carrier, at least a portion of the first anchor (442) and the second anchor (452) extending laterally and outwardly away from the base portion (430), the first anchor (442) and second anchor (452) being configured to move relative to the base portion (430) to engage tissue at a biopsy site, the base portion (430) defining a plurality of curved sections disposed between the first anchor (445), the second anchor (452) and a curved proximal end portion (436), the plurality of curved sections being configured to define a distinct appearance under x-ray visualization, **characterized by**
the base portion defining a first wire portion (432) and a second wire portion (434), the first wire portion (432) and second wire portion (434) being bent in opposite directions such that the first wire portion (432) and the second wire portion (434) cross one another at two or more points to define at least a portion of a curved section of the plurality of curved sections.

2. The marker of claim 1, the first anchor (442) and the second anchor (452) extending laterally from the base portion (430) at an angle relative to a longitudinal axis defined by the carrier.

3. The marker of claim 2, the plurality of curved sections being oriented along the longitudinal axis defined by the carrier.

4. The marker of any of claims 1 through 3, the carrier including a hydrogel marker material, the hydrogel marker material being configured to expand in the presence of moisture, the first anchor (442) and second anchor (452) being configured to respond to expansion of the hydrogel marker material to increase engagement of the first anchor (442) and the second anchor (452) with tissue.

5. The marker of any of claims 1 through 4, the first anchor (442) and the second anchor (452) each including an anchor member (446, 456), the anchor member (446, 456) being disposed on an outer end of each of the first anchor (442) and the second anchor (452).

6. The marker of claim 5, the anchor member (446, 456) including one or more coils (446, 456).

7. The marker of claim 5, the anchor member (446, 456) defining a plurality of ridges.

8. The marker of claim 1, the first wire portion (432) and the second wire portion (434) each defining a straight section, the first wire portion (432) and the second wire portion (434) extending parallel to one another within the straight section.

9. The marker of claims 1 or 8, the first wire portion (432) and the second wire portion (434) being joined at the proximal end portion (436), the proximal end portion (436) defining an end coil defining one or more wire loops.

10. The marker of claims 1 or 8, the first wire portion (432) and the second wire portion (434) extending from the first anchor (442) and the second anchor (563) toward the proximal end portion (436), the first wire portion (432) and the second wire portion (434) curving away from each other at the proximal end portion (436) such that the first wire portion (432) and the second wire portion (434) are separate discrete elements.

## Patentansprüche

1. Biopsiestellenmarker, umfassend:
einen Träger; und
ein Markerelement (420), das einen Basisabschnitt (430), einen ersten Anker (442) und einen zweiten Anker (452) einschließt, wobei der Basisabschnitt (430) mindestens teilweise innerhalb des Trägers angeordnet ist, wobei sich mindestens ein Abschnitt des ersten Ankers (442) und des zweiten Ankers (452) seitlich und nach außen von dem Basisabschnitt (430) weg erstreckt, wobei der erste Anker (442) und der zweite Anker (452) dazu ausgebildet sind, sich relativ zu dem Basisabschnitt (430) zu bewegen, um mit Gewebe an einer Biopsiestelle in Eingriff zu gelangen, wobei der Basisabschnitt (430) eine Vielzahl von gekrümmten Bereichen definiert, die zwischen dem ersten Anker (445), dem zweiten Anker (452) und einem gekrümmten proximalen Endabschnitt (436) angeordnet sind, wobei die Vielzahl von gekrümmten Bereichen dazu ausgebildet ist, unter Röntgenbildgebung ein markantes Erscheinungsbild zu definieren, **dadurch gekennzeichnet, dass**
der Basisabschnitt einen ersten Drahtabschnitt (432) und einen zweiten Drahtabschnitt (434) definiert, wobei der erste Drahtabschnitt (432) und der zweite Drahtabschnitt (434) in entgegengesetzte Richtungen gebogen sind, so dass der erste Drahtabschnitt (432) und der zweite Drahtabschnitt (434) einander an zwei oder mehr Punkten kreuzen, um mindestens einen Abschnitt eines gekrümmten Bereichs der Vielzahl von gekrümmten Bereichen zu definieren.

2. Marker nach Anspruch 1, wobei sich der erste Anker (442) und der zweite Anker (452) seitlich von dem Basisabschnitt (430) in einem Winkel relativ zu einer durch den Träger definierten Längsachse erstrecken.

3. Marker nach Anspruch 2, wobei die Vielzahl von gekrümmten Bereichen entlang der durch den Träger definierten Längsachse orientiert ist.

4. Marker nach einem der Ansprüche 1 bis 3, wobei der Träger ein Hydrogel-Markermaterial einschließt, wobei das Hydrogel-Markermaterial dazu ausgebildet ist, sich in Gegenwart von Feuchtigkeit auszudehnen, wobei der erste Anker (442) und der zweite Anker (452) dazu ausgebildet sind, auf eine Ausdehnung des Hydrogel-Markermaterials zu reagieren, um den Eingriff des ersten Ankers (442) und des zweiten Ankers (452) mit Gewebe zu erhöhen.

5. Marker nach einem der Ansprüche 1 bis 4, wobei der erste Anker (442) und der zweite Anker (452) jeweils ein Ankerelement (446, 456) einschließen, wobei das Ankerelement (446, 456) an einem äußeren Ende jedes von dem ersten Anker (442) und dem zweiten Anker (452) angeordnet ist.

6. Marker nach Anspruch 5, wobei das Ankerelement (446, 456) eine oder mehrere Windungen (446, 456) einschließt.

7. Marker nach Anspruch 5, wobei das Ankerelement (446, 456) eine Vielzahl von Rippen definiert.

8. Marker nach Anspruch 1, wobei der erste Drahtabschnitt (432) und der zweite Drahtabschnitt (434) jeweils einen geradlinigen Bereich definieren, wobei sich der erste Drahtabschnitt (432) und der zweite Drahtabschnitt (434) innerhalb des geradlinigen Bereichs parallel zueinander erstrecken.

9. Marker nach Anspruch 1 oder 8, wobei der erste Drahtabschnitt (432) und der zweite Drahtabschnitt (434) an dem proximalen Endabschnitt (436) verbunden sind, wobei der proximale Endabschnitt (436) eine Endwindung definiert, die eine oder mehr Drahtschleifen definiert.

10. Marker nach Anspruch 1 oder 8, wobei sich der erste Drahtabschnitt (432) und der zweite Drahtabschnitt (434) von dem ersten Anker (442) und dem zweiten Anker (563) zu dem proximalen Endabschnitt (436) hin erstrecken, wobei sich der erste Drahtabschnitt (432) und der zweite Drahtabschnitt (434) an dem proximalen Endabschnitt (436) voneinander weg krümmen, so dass der erste Drahtabschnitt (432) und der zweite Drahtabschnitt (434) separate diskrete Elemente sind.

## Revendications

1. Marqueur de site de biopsie, comprenant :
un support ; et
un élément (420) marqueur incluant une partie (430) de base, un premier ancrage (442), et un deuxième ancrage (452),
la partie (430) de base étant au moins partiellement disposée à l'intérieur du support, au moins une partie du premier ancrage (442) et du deuxième ancrage (452) s'étendant latéralement et vers l'extérieur à l'écart de la partie (430) de base, le premier ancrage (442) et le deuxième ancrage (452) étant configurés pour se déplacer par rapport à la partie (430) de base de manière à venir en prise avec un tissu au niveau d'un site de biopsie, la partie (430) de base définissant une pluralité de sections incurvées disposées entre le premier ancrage (445), le deuxième ancrage (452) et une partie (436) d'extrémité proximale incurvée, la pluralité de sections incurvées étant configurées pour définir un aspect distinct sous visualisation par rayons X, **caractérisé en ce que**
la partie de base définit une première partie (432) de fil et une deuxième partie (434) de fil, la première partie (432) de fil et la deuxième partie (434) de fil étant courbées dans des directions opposées de telle sorte que la première partie (432) de fil et la deuxième partie (434) de fil se croisent l'une l'autre en deux points ou plus pour définir au moins une partie d'une section incurvée de la pluralité de sections incurvées.

2. Marqueur selon la revendication 1, le premier ancrage (442) et le deuxième ancrage (452) s'étendant latéralement à partir de la partie (430) de base selon un angle par rapport à un axe longitudinal défini par le support.

3. Marqueur selon la revendication 2, la pluralité de sections incurvées étant orientées le long de l'axe longitudinal défini par le support.

4. Marqueur selon l'une quelconque des revendications 1 à 3, le support incluant un matériau marqueur d'hydrogel, le matériau marqueur d'hydrogel étant configuré pour se dilater en présence d'humidité, le premier ancrage (442) et le deuxième ancrage (452) étant configurés pour répondre à une dilatation du matériau marqueur d'hydrogel de manière à augmenter la prise du premier ancrage (442) et du deuxième ancrage (452) avec le tissu.

5. Marqueur selon l'une quelconque des revendications 1 à 4, le premier ancrage (442) et le deuxième ancrage (452) incluant chacun un organe (446, 456) d'ancrage, l'organe (446, 456) d'ancrage étant disposé sur une extrémité extérieure de chacun du premier ancrage (442) et du deuxième ancrage (452).

6. Marqueur selon la revendication 5, l'organe (446, 456) d'ancrage incluant une ou plusieurs spires (446, 456).

7. Marqueur selon la revendication 5, l'organe (446, 456) d'ancrage définissant une pluralité d'arêtes.

8. Marqueur selon la revendication 1, la première partie (432) de fil et la deuxième partie (434) de fil définissant chacune une section droite, la première partie (432) de fil et la deuxième partie (434) de fil s'étendant parallèlement l'une à l'autre à l'intérieur de la section droite.

9. Marqueur selon la revendication 1 ou la revendication 8, la première partie (432) de fil et la deuxième partie (434) de fil étant jointes au niveau de la partie (436) d'extrémité proximale, la partie (436) d'extrémité proximale définissant une spire d'extrémité définissant une ou plusieurs boucles de fil.

10. Marqueur selon la revendication 1 ou la revendication 8, la première partie (432) de fil et la deuxième partie (434) de fil s'étendant à partir du premier ancrage (442) et du deuxième ancrage (563) vers la partie (436) d'extrémité proximale, la première partie (432) de fil et la deuxième partie (434) de fil s'incurvant à l'écart l'une de l'autre au niveau de la partie (436) d'extrémité proximale de telle sorte que la première partie (432) de fil et la deuxième partie (434) de fil sont des éléments discrets séparés.
